# FASCICULE DE BREVET EUROPEEN

(11) **EP 0 964 709 B1**
(45) Date de publication et mention de la délivrance du brevet: **02.05.2002**
(21) Numéro de dépôt: 98906979.4
(22) Date de dépôt: 05.02.1998
(51) Int. Cl.: A61L 31/00

(54) **MATERIAU COLLAGENIQUE UTILE NOTAMMENT POUR LA PREVENTION D'ADHERENCES POST-OPERATOIRES**
COLLAGENMATERIAL GEEIGNET FÜR DIE HEMMUNG DER POSTOPERATIVEN ADHÄSION
COLLAGENIC MATERIAL USEFUL IN PARTICULAR FOR PREVENTING POST-OPERATIVE ADHESIONS

(30) Priorité: 06.02.1997 FR 9701373; 17.09.1997 FR 9711589
(43) Date de publication de la demande: 22.12.1999
(73) Titulaire: Imedex Biomateriaux, 69630 Chaponost (FR)
(72) Inventeur: TAYOT, Jean-Louis, F-69890 La Tour de Salvagny (FR); TARDY, Michel, F-69005 Lyon (FR); GRAVAGNA, Philippe, F-69540 Irigny (FR)
(74) Mandataire: Bernasconi, Jean Raymond
(86) Numéro de dépôt international: FR9800214
(87) Numéro de publication internationale: WO9834656

(56) Documents cités:
- EP-A- 0 213 563
- EP-A- 0 664 132
- WO-A-96/08277

## Description

La présente invention concerne un matériau collagénique biocompatible, non toxique, potentiellement adhérent et rapidement biodégradable. Elle concerne plus particulièrement un matériau collagénique capable d'inhiber la formation d'adhérences post-opératoires.

L'invention est également relative à un procédé d'obtention d'un tel matériau.

Les adhérences post-opératoires se développent après intervention chirurgicale chez un patient dont certains organes ont subi un traumatisme engendré par l'acte chirurgical lui-même.

Les plaies créées par dissection d'organes et de tissus, ou toute autre intervention se caractérisent par deux phénomènes :
- ces plaies ne sont pas étanches et laissent exsuder spontanément un liquide plasmatique contenant de la fibrine, quelquefois même du sang si l'hémostase n'a pu être achevée de manière parfaite ; certains organes laissent suinter d'autres liquides comme la bile du foie ou le liquide cérébro-spinal au niveau du système nerveux.
- les surfaces lésées ne possèdent plus de barrière tissulaire organisée et stable et quelquefois même sont parsemées de corps étrangers : fils de sutures, agrafes, caillots, agents infectieux, tissus brûlés par les bistouris électriques ou faisceaux lasers.

Ces deux caractéristiques entraînent une réaction de l'organisme commençant par l'inflammation et se poursuivant par une migration et une prolifération cellulaires intenses, généralement mal organisées. Celles-ci créent le plus souvent des tissus néoformés, fibreux, vascularisés, reliant les organes lésés aux organes voisins.

De nombreuses revues ont été publiées sur le sujet (voir en particulier Dl ZEREGA (1994)).

Diverses solutions ont déjà été proposées, notamment sur le plan pharmacologique, avec plus ou moins de succès.

La solution la plus efficace que l'on connaisse à ce jour est de poser sur la plaie une barrière physique, isolant les organes les uns des autres et leur permettant de cicatriser de manière indépendante sans développer d'interrelations.

Les barrières physiques qui conduisent aux meilleurs résultats sont constituées de tissus artificiels, non dégradables, sur lesquels les cellules ne s'attachent pas. Le Téflon ® et le silicone sont parmi les polymères les plus efficaces.

L'inconvénient majeur est qu'il faut retirer ces barrières physiques plusieurs semaines plus tard, par une seconde opération chirurgicale qui peut, en outre, créer elle-même d'autres adhérences secondaires au niveau de la laparotomie.

Il est donc indispensable de développer des barrières biodégradables qui évitent une seconde intervention.

Dans cet objectif, de nombreux polymères naturels biodégradables ont déjà été proposés, à partir de gélatine, de collagène, de polysaccharides, de mucopolysaccharides, etc. mais ceux-ci n'ont pas conduit à des résultats satisfaisants.

Selon l'équipe de RODEHEAVER (HARRIS et Coll., 1995) l'objectif minimum est que la barrière demeure présente au moins pendant 36 heures. Ceci s'explique en raison du fait qu'un temps minimum est nécessaire pour permettre les mécanismes complexes de cicatrisation.

Cependant, le temps de résorption ne doit pas être trop long. Si le matériau demeure plusieurs semaines au contact de la plaie, il peut entraîner des réactions inflammatoires persistantes qui favorisent une réaction fibreuse inorganisée et plus importante, et qui peuvent causer localement des problèmes anatomiques : épaisseur, rigidité, rétrécissement, ischémie, granulome ou un foyer persistant d'infection surtout en contact avec les intestins et les organes digestifs.

En outre, il apparaît que le matériau devant servir de barrière physique devrait adhérer correctement aux tissus lésés, surtout pour la protection d'organes mobiles ou soumis à des distensions variables comme l'intestin, ou plus simplement pour éviter de migrer en réponse aux mouvements du patient et aux contraintes mécaniques que ceux-ci entraînent.

La difficulté d'obtenir toutes ces propriétés réunies dans un même biomatériau explique pourquoi les solutions et produits proposés à ce jour se révèlent encore très insuffisants.

Les produits biodégradables aujourd'hui commercialisés pour la prévention d'adhérences ne sont que partiellement actifs et donnent des résultats encore insuffisants.

Les dérivés de cellulose commercialisés par Johnson & Johnson Medical (Arlington, Texas, Etats-Unis), comme les produits SURGICELL ® ou INTERCEED ®, ne peuvent être utilisés en présence de sang et montrent, dans certains modèles animaux, des résultats décevants (HARRIS et Coll., 1995).

Le produit SEPRAFILM ®, commercialisé par Genzyme, (Cambridge, Massachusetts, Etats-Unis), est un film d'acide hyaluronique et de carboxyméthylcellulose qui ne semble efficace que dans la moitié des cas (BECKER et Coll., 1996). Il se dégrade très rapidement en quelques jours, mais est difficile à manipuler. Il est fragile, cassant et impossible à utiliser à travers un trocart en laparoscopie. Enfin, il perd progressivement son adhérence initiale et peut migrer à distance, laissant la plaie non protégée.

Parmi les produits récemment décrits par les chercheurs dans la littérature ou les demandes de brevet publiées, il apparaît que de nombreux problèmes restent encore non solutionnés.

Parmi ceux-ci, divers matériaux collagéniques ont été proposés.

KHOURY et Coll. (1994), pour la société COLETICA (Lyon, France), proposent une membrane de collagène constituée de deux couches, l'une à base de collagène natif, formant support et recouverte d'une seconde couche de gélatine. La conception de cette membrane s'inspire d'un patch de chirurgie viscérale développé par TAYOT et Coll. (1988). Ce matériau a une durée de dégradation très largement supérieure à un mois et ne possède donc pas la propriété essentielle qui est de disparaître très rapidement. Il présente aussi l'inconvénient de ne pas être suffisamment adhérent et en conséquence, de nécessiter des points de suture pour son application, qui peuvent engendrer des complications indésirables.

ORLY I. en 1994, pour la société COLETICA, a proposé une membrane transparente de collagène, constituée d'une seule couche ou de deux couches selon les exemples, et le plus souvent associée à une prothèse non dégradable, pour le traitement des hernies ou des éventrations. Cette membrane est constituée de collagène natif, non dénaturé. Elle doit être agrafée ou suturée. Pour faciliter l'agrafage, la transparence de la membrane est nécessaire pour éviter de blesser des zones sensibles (nerfs, vaisseaux) comme dans les hernies inguinales. Les risques associés à l'agrafage de prothèses herniaires en particulier, sont bien connus. Pour cette raison, les chirurgiens seraient davantage intéressés par le collage de ces prothèses, évitant ainsi toutes sutures ou agrafes. ORLY I. précise, de manière surprenante, que cette membrane ne doit pas être adhésive, car la membrane développée par KHOURY et Coll. dans la même société COLETICA (cité ci-dessus) ne peut être utilisée dans cette indication, en raison de ses propriétés adhésives qui ne facilitent pas l'introduction en coeliochirurgie. Le délai de résorption de cette membrane est très largement supérieur à un mois puisque, à 5 semaines, la résorption n'est pas complète et qu'il faut attendre 3 mois pour l'élimination presque totale du matériau. Malgré ces délais, très longs, le déposant affirme de manière paradoxale l'avantage d'un temps de résorption « relativement » rapide.

YEUNG et Coll., pour la société Collagen Corporation (Palo Alto, Californie, Etats-Unis) proposent, en 1995, de lier à un substrat collagénique, un agent anti-adhérences dérivé du polyéthylène glycol par liaisons covalentes. Ce produit est très complexe à réaliser et demande l'utilisation de dérivés du polyéthylène glycol difficiles à fabriquer, coûteux et non dénués de risques toxiques associés à leur réactivité chimique. Enfin, le temps de résorption indiqué pour ce matériau constitué de collagène dense est le plus souvent de 30 à 50 jours.

TARDY et Coll., en 1994 dans un brevet pour la société IMEDEX, maintenant dénommée SADUC, proposent une colle biologique liquide dérivée du collagène, qui a des propriétés anti-adhérentes. Les avantages de ce produit sont notamment son adhérence et sa dégradation rapide en quelques jours. Les propriétés adhésives viennent de la réticulation des molécules de collagène oxydé à l'acide périodique et conservé liquide à pH acide. Cette réticulation est déclenchée par addition d'une solution alcaline, ou tampon, qui neutralise le pH et transforme rapidement la solution de collagène oxydé en un solide adhérent. L'efficacité de ce produit dans la prévention d'adhérences post-opératoires, démontrée dans plusieurs modèles animaux, n'est cependant pas facilement exploitable en pratique. En effet, ce produit nécessite un stockage permanent sous forme congelée, difficile à réaliser dans les circuits hospitaliers courants.

Divers biomatériaux collagéniques obtenus par réticulation du collagène après oxydation par l'acide périodique ont été également décrits dans un brevet par M. TARDY et Coll., en 1986. Des films de collagène oxydé réticulé ont été préparés par séchage sous flux d'air stérile de solutions de collagène oxydé additionné de glycérine. Ces films ont des propriétés qui sont fonction de la quantité de glycérine utilisée. Pour obtenir des films souples, non cassants, présentant une résistance acceptable, il faut employer une quantité de glycérine suffisante, environ égale en poids à la quantité de collagène. Cependant, dans ces conditions, ils collent à leur support, aux gants et aux instruments du chirurgien, manquent de rigidité à cause de leur finesse et sont très difficiles d'emploi s'ils ne sont pas associés à un autre matériau.

L'invention décrite ci-après permet de résoudre les inconvénients cités précédemment.

Elle a en particulier pour objectif de fournir un matériau biocompatible, non toxique et non collant au toucher à l'état sec pour la facilité de manipulation mais pouvant développer des propriétés adhérentes en milieu aqueux, en particulier en milieu physiologique.

L'invention a encore pour objectif de fournir un matériau biodégradable rapidement, à savoir en moins d'un mois, et, de préférence, moins d'une semaine.

L'invention a également pour objectif de fournir un matériau collagénique adapté à une utilisation comme barrière anti-adhérences post-opératoire.

Un autre objectif de l'invention est de fournir un procédé pour l'obtention d'un tel matériau.

A cette fin, l'invention a pour objet un matériau collagénique biocompatible, non toxique et biodégradable en moins d'un mois, caractérisé en ce qu'il comprend du collagène ayant perdu au moins partiellement sa structure hélicoïdale et au moins un additif hydrophile macromoléculaire, chimiquement non réactif vis-à-vis du collagène, le collagène ayant subi un traitement de réticulation, ledit matériau n'étant pas collant au toucher à l'état sec et étant adhérant à l'état humide.

L'invention concerne également un tel matériau biodégradable en moins d'une semaine.

L'invention a également pour objet un procédé pour l'obtention d'un matériau collagénique biocompatible, non toxique, potentiellement adhérent à l'état humide et biodégradable en moins d'un mois, de préférence moins d'une semaine, caractérisé en ce qu'il comprend :
a) la préparation d'une solution de collagène;
b) le traitement de ladite solution pour faire perdre au collagène, au moins partiellement, sa structure hélicoïdale ;
c) le mélange de la solution collagénique résultante avec une solution contenant au moins un additif hydrophile macromoléculaire, chimiquement non réactif vis-à-vis du collagène présent ;
d) la réticulation dudit mélange pour obtenir le matériau collagénique désiré.

Les inventeurs ont découvert, de manière surprenante, qu'un mélange de collagène, dénaturé par chauffage modéré et d'un additif hydrophile macromoléculaire, pouvait constituer, après réticulation du collagène, un matériau biocompatible et non toxique à la fois non collant au toucher à l'état sec, adhérent en milieu aqueux (physiologique) et biodégradable en quelques jours ou quelques semaines.

Ils ont mis en évidence que l'on pouvait obtenir un tel matériau en réticulant du collagène préalablement modifié par coupure oxydative et chauffage, en présence d'un additif hydrophile macromoléculaire, chimiquement non réactif vis-à-vis du collagène.

La coupure oxydative du collagène a comme fonction de permettre une réticulation modérée ultérieure du matériau collagénique mais l'invention n'exclut pas que l'on puisse réaliser cette fonction par d'autres moyens de réticulation modérée, par exemple par rayonnement béta ou gamma, ou d'autres agents de réticulation modérée, par exemple des agents chimiques à des doses suffisamment faibles et non toxiques.

Le traitement par chauffage à température supérieure à 37°C de la solution de collagène entraîne la perte progressive de la structure hélicoïdale du collagène, mais l'invention n'exclut pas que l'on puisse réaliser cette fonction par d'autres moyens physiques ou chimiques, par exemple par ultra-sonication, ou par addition d'agents chaotropiques.

Ils ont découvert en particulier que la présence de l'additif hydrophile permettait, de manière inattendue, d'augmenter la densité et la résistance mécanique du collagène, de le rendre potentiellement adhésif, c'est-à-dire de permettre une adhésivité satisfaisante, notamment en milieu physiologique, à des organes et de favoriser sa dégradation, qui peut ainsi être accomplie, suivant les formes de réalisation de l'invention, en moins de sept jours ou moins de 4 semaines.

On décrit ci-après le procédé de préparation d'un matériau collagénique selon la présente invention.

Le collagène mis en oeuvre pour l'obtention d'un matériau collagénique tel que défini précédemment, peut être indifféremment d'origine animale ou humaine ou obtenu par des moyens de recombinaison génétique. On utilise de préférence du collagène natif, solubilisé à pH acide ou après traitement par digestion à la pepsine. Il peut s'agir en particulier du collagène bovin de type I ou de collagènes humains de type I ou III ou encore de mélanges en toutes proportions de ces derniers.

Selon un mode de réalisation de l'invention, le collagène est modifié par coupure oxydative. On peut utiliser à cet effet de l'acide périodique ou l'un de ses sels selon la technique décrite par M. TARDY et Coll. (1986).

On rappelle brièvement que cette technique consiste à soumettre une solution acide de collagène à l'action de l'acide périodique ou l'un de ses sels par mélange avec une solution de cet acide ou sel à une concentration comprise entre 1 et 10⁻⁵ M, de préférence entre 5 10⁻³ M et 10⁻¹ M, à une température voisine de la température ambiante, pendant une durée pouvant aller de 10 minutes à 72 heures.

Selon l'invention, on utilise une solution acide de collagène dont la concentration est comprise entre 5 et 50 g/l. La concentration en collagène est de préférence 30 g/l.

Ce traitement provoque des coupures dans certains constituants du collagène qui sont l'hydroxyproline et les sucres, et crée ainsi des sites réactifs sans en provoquer la réticulation.

Le collagène oxydé ainsi préparé en solution, est chauffé à une température supérieure à 37°C, de préférence à une température comprise entre 40 et 50° C. Il en résulte que la structure hélicoïdale du collagène est dénaturée, au moins partiellement.

On réalise ensuite la réticulation du collagène en présence d'au moins un additif hydrophile macromoléculaire, chimiquement non réactif vis-à-vis du collagène.

Par « chimiquement non réactif vis-à-vis du collagène », on entend un composé hydrophile qui n'est pas susceptible de réagir avec le collagène présent, notamment qui ne forme pas de liaison covalente avec celui-ci lors de sa réticulation.

L'additif hydrophile macromoléculaire selon l'invention présente un poids moléculaire avantageusement supérieur à 3 000 daltons,

Il peut s'agir de polymères hydrophiles de synthèse, avantageusement de poids moléculaire compris entre 3 000 et 20 000 Daltons. Le polyéthylèneglycol est particulièrement préféré.

Il peut s'agir également de polysaccharides, parmi lesquels on peut citer l'amidon, le dextrane et la cellulose qui sont préférés.

On peut également prévoir l'utilisation de tels polysaccharides sous forme oxydée faisant apparaître des fonctions carboxyliques dans ces molécules.

Les mucopolysaccharides peuvent également convenir aux fins de l'invention mais ne sont pas préférés car leur origine animale particulière les rend difficiles à préparer en satisfaisant aux normes réglementaires de traçabilité.

L'additif hydrophile est sélectionné en fonction de divers paramètres liés notamment à son application, comme son prix, son innocuité, sa biodégradabilité et/ou sa facilité d'élimination, notamment par voie rénale, en cas d'application thérapeutique.

Ladite réticulation est réalisée en mélangeant, à pH neutre, une solution de collagène modifié par coupure oxydative et chauffage comme indiqué ci-dessus, avec une solution contenant au moins un additif hydrophile macromoléculaire.

La concentration en additif(s) hydrophile(s) est de 2 à 10 fois inférieure à celle du collagène.

La réticulation du collagène en présence de l'additif hydrophile est réalisée à une température comprise entre 4 et 30° C, de préférence 18 à 25° C.

Selon une variante de mise en oeuvre, on prévoit d'effectuer la réticulation en présence de glycérine qui peut être ajoutée au mélange collagène/additif hydrophile. Dans ce cas, la concentration en glycérine est avantageusement comprise entre 3 et 8 g/l, ne dépassant pas le tiers de la concentration du collagène.

La polymérisation s'effectue pendant le séchage du matériau.

Selon l'invention, on pense, sans que cela puisse être considéré comme limitatif, que l'additif hydrophile qui est présent lors de la réticulation du collagène, est contenu dans le réseau collagénique formé, bien qu'il ne réagisse pas lui-même avec le collagène.

Selon les applications envisagées, le matériau collagénique réticulé peut être soumis à divers traitements classiques tels que séchage, stérilisation, etc.

Le collagène réticulé peut être séché notamment dans un flux d'air stérile, si nécessaire.

La stérilisation est avantageusement opérée par irradiation avec des rayonnements béta (irradiation électronique) ou gamma (irradiation par le cobalt radioactif).

Conformément à l'invention, le matériau collagénique peut être préparé sous la forme d'un film, gel ou pâte.

Selon les applications envisagées, on prévoit avantageusement le matériau collagénique sous la forme d'un film.

Selon un mode de réalisation particulièrement préféré, la solution contenant le collagène ayant perdu au moins partiellement sa structure hélicoïdale notamment par chauffage, et éventuellement modifié par coupure oxydative, un additif hydrophile macromoléculaire, et éventuellement de la glycérine, est répartie uniformément sur un support inerte sensiblement plan, pour former un film réticulé.

Le support est inerte en ce qu'il ne réagit pas avec les composés précités et n'intervient pas dans le processus de réticulation. On peut utiliser un support hydrophobe de type PVC ou polystyrène.

Dans ce cas, on applique sur le support une solution dans laquelle les concentrations en collagène, en additif hydrophile et en glycérine, si elle est présente, sont de préférence, respectivement comprises entre 2 et 6 % pour le collagène, 0,6 et 2 % pour l'additif hydrophile, 0,3 et 0,8 % pour la glycérine.

La couche mince appliquée présente avantageusement une densité allant de 0,05 à 0,3 g/cm².

Après réaction, le film est séparé du support.

Le matériau collagénique selon l'invention contient du collagène et au moins un additif hydrophile macromoléculaire selon un rapport collagène/additif(s) hydrophile(s) de 1/1 à 9/1, de préférence de 2/1 à 4/1 et plus préférentiellement encore de 3/1.

Il est stable à température ambiante, et reste stable pendant un temps suffisant pour sa manipulation, en milieu aqueux à des températures allant jusqu'à 37 à 40° C.

Le matériau collagénique selon l'invention est utile pour la prévention des adhérences post-opératoires.

Il peut être préparé sous forme « prête à l'emploi » comme par exemple par découpage aux dimensions appropriées à l'application envisagée, d'un film tel que décrit ci-dessus et conditionnement dans des conditions stériles.

Lorsqu'il est préparé sous forme de film, il est particulièrement adapté à cette application, notamment pour simplifier et accélérer le geste chirurgical.

En effet, le film collagénique obtenu est de manipulation aisée car il ne colle pas au toucher à l'état sec et il ne colle pas aux instruments.

Il présente par ailleurs une densité et une résistance mécanique accrues tout en étant relativement souple comme nécessaire.

La glycérine permet d'améliorer la souplesse du matériau final obtenu, et peut ainsi en faciliter l'utilisation.

Le matériau collagénique selon l'invention est capable de développer, en milieu aqueux, des propriétés d'adhésion.

Après implantation chez un patient, il peut adhérer de manière suffisante sur l'organe à protéger ce qui lui permet de tenir en place et est de ce fait adapté à une utilisation comme barrière anti-adhérences post-opératoire. En effet, au contact des tissus, un appel d'eau tissulaire entraîne une adhésion immédiate du film collagénique. L'appel d'eau gonfle progressivement le film de collagène dont la réticulation est assez peu dense pour permettre une certaine mobilité et souplesse des chaînes le constituant.

Il se forme ainsi en quelques minutes à quelques heures, un gel de collagène oxydé, réticulé et très hydraté grâce à l'additif hydrophile emprisonné dans les mailles du gel.

Ce gel acquiert des propriétés adhésives suffisantes pour établir une adhésion satisfaisante du biomatériau sur la plaie à protéger.

En outre, l'additif hydrophile macromoléculaire disparaît par diffusion à travers le matériau collagénique oxydé et réticulé, en quelques jours, matériau dont le gonflement favorise la dégradation en moins d'un mois, voire moins de 7 jours, notamment en 2 à 3 jours.

Si on augmente le taux de réticulation du matériau collagénique, notamment par incubation à 37°C en milieu humide, avant la stérilisation par irradiation, on peut augmenter le temps de résorption jusqu'à 2-4 semaines.

Ce temps de résorption compris entre 1 et 4 semaines est intéressant pour certaines applications où la cicatrisation des tissus lésés est un phénomène plus lent.

L'invention sera décrite plus en détail à l'aide des exemples donnés ci-après à titre illustratif et non limitatif.

### EXEMPLES

### Exemple 1:

### Préparation d'un précipité acide de collagène modifié par coupure oxydative à l'aide d'acide périodique et non réticulé.

Cet exemple est directement réalisé à partir du brevet précédemment cité de TARDY et Coll. (1994).

Le collagène utilisé est du collagène bovin de type I, extrait de derme de veau par solubilisation à pH acide ou par digestion à la pepsine et purifié par précipitations salines selon des techniques déjà décrites.

Les produits commercialisés par la société COLLAGEN Corp., sous les noms de VITROGEN ® ou ZYDERM ®, peuvent convenir dans cette application.

On utilise de préférence des fibres sèches de collagène, obtenues par précipitation d'une solution acide de collagène par addition de NaCI, puis lavages et séchage du précipité obtenu par des solutions aqueuses d'acétone de concentration croissante de 80 % à 100 %.

On peut, de la même manière, utiliser des collagènes humains de type I ou III ou leur mélange en toutes proportions.

Une solution de collagène à 30 g/l est préparée par dissolution en HCI 0,01 N. Son volume est de 49 litres. Elle est additionnée d'acide périodique à une concentration finale de 8 mM soit 1,83 g/l.

On réalise l'oxydation à température ambiante voisine de 22°C, pendant 3 heures à l'abri de la lumière.

Puis, la solution est additionnée d'un volume égal d'une solution de chlorure de sodium pour obtenir une concentration finale de 41 g/l NaCI.

Après 30 minutes d'attente, le précipité est récolté par décantation à travers un tamis de toile de porosité voisine de 100 microns, puis lavé 4 fois avec une solution de NaCI 41 g/l en HCI 0,01 N. On obtient 19 kg. de précipité salin acide. Ces lavages permettent l'élimination de toutes traces d'acide périodique ou dérivés iodés formés pendant l'oxydation du collagène.

Ensuite, plusieurs lavages en solution aqueuse d'acétone à 80 % permettent la concentration du précipité de collagène et l'élimination des sels présents.

Un lavage final en acétone 100 % permet de préparer 3,6 kg d'un précipité acétonique très dense, de collagène oxydé acide, non réticulé, ne contenant aucune autre trace de produit chimique indésirable.

### Exemple 2 :

### Préparation de films collagéniques anti-adhérences post-opératoires selon l'invention.

La pâte acétonique préparée selon l'exemple 1 est reprise en eau distillée apyrogène à 40°C, pour obtenir une concentration de collagène de 3 %.

La solution de 44 litres est chauffée 30 minutes à 50°C, puis filtrée stérilement sur membrane de porosité 0,45 microns dans une étuve à 40°C.

Cette solution est additionnée à 30°C d'une solution stérile concentrée de PEG 6 000 (polyéthylène-glycol de poids moléculaire 6 000 Daltons) pour atteindre une concentration de 1 % en PEG, puis' de glycérine pour atteindre une concentration de 0,6 % en glycérine. Le pH de la solution est ajusté à 7,0 par addition d'une solution concentrée de soude NaOH.

La solution est ensuite ajustée en volume par de l'eau stérile pour atteindre une concentration finale de 2,7 % de collagène, 0,9 % PEG et 0,54 % de glycérine, puis répartie en couche mince à une densité de 0,133 g/cm² sur un support plan hydrophobe de type PVC ou polystyrène. Les surfaces sont ensuite soumises à un flux d'air stérile à température ambiante, qui conduit à l'évaporation en 18 heures environ.

Le film obtenu se décolle très facilement du support. Il peut être découpé facilement aux dimensions souhaitées pour l'expérimentation.

Ensuite, pour suivre la réglementation pharmaceutique, et améliorer la stabilité au stockage, le film est inclus dans un double sachet non perméable à l'air.

L'ensemble est stérilisé par irradiation béta et reçoit une dose supérieure ou égale à 25 kilogreys.

En général, le film séché contient encore de l'eau résiduelle jusqu'à une concentration pouvant atteindre 20 %.

Une composition typique dans l'exemple considéré est de 60 % en poids de collagène, 20 % en poids de PEG, 12 % en poids de glycérine et 8 % en poids d'eau.

Le film obtenu est stable à température ambiante. Il reste stable et facilement manipulable après 2 heures d'incubation en eau à 37 °C.

La présence de glycérine dans le matériau n'est pas indispensable ; elle améliore la souplesse du film et en facilite l'utilisation.

Cet exemple peut être réalisé en remplaçant le PEG 6 000 par du PEG 3 000 ou 4 000, de l'amidon soluble (catalogue OSI France, référence A2620) ou par du Dextran T40 (catalogue PHARMACIA Fine Chemicals, Uppsala, Suède) ou par de la carboxymethylcellulose. Les concentrations utilisées et le mode opératoire sont identiques.

### ETUDE DES PROPRIETES DU FILM COLLAGENIQUE SELON L'INVENTION DANS LA PREVENTION DES ADHERENCES POST-OPERATOIRES.

### • Toxicologie

Le film collagène-PEG réalisé selon l'exemple 2 (ou un broyât de ce film) donne des résultats satisfaisants dans les tests toxicologiques standardisés auxquels sont soumis les biomatériaux pour implantation :
- absence de caractère mutagène dans le test d'AMES
- réaction normale, modérée dans le test d'hypersensibilité retardée sur cobaye (réaction de classe III)
- absence de toxicité systémique par voie intraveineuse ou intrapéritonéale chez la souris et le rat
- dégradation :
   . en moins de 7 jours par voie sous cutanée chez le rat
   . en 2 à 3 jours par voie intrapéritonéale chez le rat.

### • Propriétés anti-adhérences post-opératoires

Les propriétés anti-adhérences post-opératoires ont été analysées selon le protocole décrit en référence par HARRIS et Coll. (1995).

Le protocole décrit dans cette publication a été mis en oeuvre sur des groupes de 10 rats.

Les essais consistent à créer une abrasion et une déshydratation sur des surfaces de 2 cm² de paroi péritonéale et de caecum, au contact l'une de l'autre.

Le groupe de rat témoins ne reçoit aucun produit de protection des plaies ainsi crées. Il est comparé au groupe de rats qui reçoit un film collagène-PEG selon l'exemple 2, recouvrant complètement les plaies, en débordant de 5 mm sur les parties externes non abrasées.

Après 7 jours d'attente, conformément au protocole publié, les résultats sont nets :
- Aucune adhérence n'est observée entre les deux surfaces lésées, dans le groupe de rats traités par le film de l'invention et la cicatrisation de chaque plaie initiale est complète.
- Toute trace du film a disparu.

Le groupe de rats témoins, non traité par le film de l'invention, montre des adhérences pour chacun des 10 rats, dont les caractéristiques sont identiques aux résultats publiés dans le document HARRIS et Coll. (1995) mentionné ci-dessus.

En testant les films biodégradables réalisés selon l'exemple 2 dans d'autres protocoles opératoires sur d'autres modèles animaux (WISEMANN 1992 et 1994) l'efficacité anti-adhérences a pu être démontrée dans des applications en neurochirurgie (notamment hernies discales, laminectomies vertébrales), en chirurgie cardiaque et en gynécologie, notamment chirurgie utérine. L'invention a donc également pour objet l'utilisation des matériaux selon l'invention dans ces indications chirurgicales, ainsi que dans la chirurgie orthopédique (notamment au niveau des tendons) et la chirurgie ophtalmologique.

### Exemple 3 :

### Préparation d'un film collagénique selon un autre mode de réalisation de l'invention.

On prépare un film comme décrit à l'exemple 2.

Après séparation de son support et découpage, chaque film est conditionné individuellement en sachet primaire perméable à la vapeur, puis incubé 12 heures dans une étuve à 37°C dont le taux d'humidité relative est supérieur à 80 %.

Ensuite le sachet précédent est inclus dans un conditionnement secondaire non perméable à l'air et est stérilisé comme à l'exemple 2.

La dégradation du matériau s'effectue en 2 à 4 semaines par implantation sous cutanée ou intrapéritonéale chez le rat.

Le film selon cet exemple est dégradé moins rapidement dans l'organisme que le film de l'exemple 2. On constate qu'il est moins adhérent à la plaie tissulaire et qu'il gonfle moins en milieu physiologique. De ce fait, un tel matériau sera de préférence indiqué dans des applications chirurgicales où le risque de déplacement du matériau est faible. Pour les autres applications, il sera préférable de le fixer par des moyens appropriés (colle, suture, etc.). Dans ces conditions les mêmes propriétés anti-adhérences sont observées in vivo.

### REFERENCES

1. Dl ZEREGA G.S. (1994) - « Contemporary adhesion prevention », *Fertility and Sterility.* 61 (2), 219-235.
2. HARRIS E.S. - MORGAN R.F. - RODEHEAVER G.T. (1995) - « Analysis of the kinetics of peritoneal adhesion formation in the rat and evaluation of potential antiadhesive agents », *Surgery*, 117 (6), 663-669.
3. BECKER J.M. - DAYTON M.T. - FAZIO V.W. - BECK D.E. - STRYKER S.J. - WEXNER S.D. - WOLFF B.G. - ROBERTS P.L. - SMITH L.E. - SWEENEY S.A. - MOORE M. (1996) - « Prevention of postoperative abdominal adhesions by a sodium hyaluronate-based bioresorbable membrane : A prospective, randomized, double-blind multicenter study », *The J*. *of Amer. Collège of Surgeons,* 183 (4), 297-306.
4. KHOURY W. - ABDUL-MALAK N. - HUC A. (1994) - « Membrane collagénique anti-adhérence post-opératoire », *Demande de brevet en France N° 94 06995 du 08.06.94 (publiée sous le N° 2 720 945)..*
5. TAYOT J.L. - MARESCAUX J. - DUMAS H. - TARDY M. (1988) - « Visceral surgery patch », *Brevet US-A-5 201 745 délivré le 13 avril 1993 (sous priorité de la demande de brevet français N° 88 03321 du 15.03.88, délivrée le 13.07.90 sous le N° 2 628 634).*
6. ORLY I. (1994) - « Use of collagen membranes as peritoneal renewing prostheses », *Demande de brevet internationale WO 96*/*082 77 (sous priorité de la demande de brevet français N° 94 11 015 du 15.09.94).*
7. YEUNG J.E. - CHU G.H. - DE LUSTRO F.A. - RHEE W.M. (1995) - « Anti adhesion films and compositions for medical use », *Demande de brevet européen N° 96 102 339.7 (publiée sous le N° 0 732 110) (sous priorité de la demande de brevet américain Serial N° 403 360 du 14.03.95).*
8. TARDY M. - TIOLLIER J. - TAYOT J.L. (1994) - « Composition adhésive, à usage chirurgical, à base de collagène modifié par coupure oxydative et non réticulé » - *Demande de brevet français N° 94 00715. du 24.01.94, délivrée le 02.08.96 sous le N° 2 715 309.*
9. TARDY M. - TAYOT J.L. (1986) - « Process for the treatment of collagen, notably for facilitating its cross-linking, and the collagen obtained by the application of the said process », *Brevet US-A-4 931 546 délivré le 05*/*06*/*1990 (sous priorité de la demande de brevet français N° 86 10 160 du 11.07.86*, *délivrée le 12.05.89 sous le N° 2 601 371).*
10. WISEMAN, D. « Polymers for the prevention of surgical adhesions » in Polymeric Site-specific pharmaco-therapy, Ed. A.J. DOMB, John Wiley & Sons Ltd, 1994.
11. WISEMAN, D. et al., « Fibrinolytic drugs prevent pericardial adhesions in the rabbit » J. of Surgical Research 53, 362-368, 1992.

## Revendications

1. Matériau collagénique biocompatible, non toxique et biodégradable en moins d'un mois, **caractérisé en ce qu'**il comprend du collagène et au moins un additif hydrophile macrombléculaire, chimiquement non réactif vis-à-vis du collagène, le collagène ayant perdu au moins partiellement sa structure hélicoïdale et ayant subi un traitement de réticulation, ledit matériau n'étant pas collant au toucher à l'état sec et étant adhérent à l'état humide.

2. Matériau collagénique selon la revendication 1, **caractérisé en ce qu'**il est biodégradable en moins d'une semaine.

3. Matériau collagénique selon l'une des revendications 1 et 2, **caractérisé en ce que** le collagène a été modifié par chauffage modéré au-dessus de 37°C.

4. Matériau collagénique selon l'une des revendications 1 à 3, **caractérisé en ce qu'**il comprend du collagène modifié par coupure oxydative réticulé en présence d'au moins un additif hydrophile macromoléculaire chimiquement non réactif vis-à-vis dudit collagène.

5. Matériau collagénique selon la revendication 4, **caractérisé en ce que** le collagène est modifié par coupure oxydative à l'aide d'acide périodique ou l'un de ses sels.

6. Matériau collagénique selon l'une des revendications 1 à 3, **caractérisé en ce qu'**il est réticulé par irradiation béta ou gamma en présence d'un additif hydrophile macromoléculaire chimiquement non réactif vis-à-vis du collagène.

7. Matériau collagénique selon l'une quelconque des revendications précédentes, **caractérisé en ce que** l'additif hydrophile macromoléculaire présente un poids moléculaire supérieur à 3 000 Daltons.

8. Matériau collagénique selon la revendication 7, **caractérisé en ce que** l'additif hydrophile est un polymère hydrophile de poids moléculaire compris entre 3 000 et 20 000 Daltons.

9. Matériau collagénique selon l'une quelconque des revendications précédentes, **caractérisé en ce que** l'additif hydrophile est le polyéthylèneglycol.

10. Matériau collagénique selon l'une quelconque des revendications 1 à 7, **caractérisé en ce que** l'additif hydrophile est un polysaccharide choisi de préférence parmi l'amidon, le Dextran et la cellulose.

11. Matériau collagénique selon l'une quelconque des revendications précédentes, **caractérisé en ce qu'**il comprend du collagène et au moins un additif hydrophile macromoléculaire selon un rapport collagène/additif(s) hydrophile(s) allant de 1/1 à 9/1, de préférence de 2/1 à 4/1 et plus préférentiellement encore de 3/1.

12. Matériau collagénique selon l'une quelconque des revendications précédentes, **caractérisé en ce qu'**il contient en outre de la glycérine.

13. Matériau collagénique selon l'une quelconque des revendications précédentes, **caractérisé en ce qu'**il se présente sous la forme d'un film.

14. Matériau collagénique selon l'une quelconque des revendications 1 à 13, **caractérisé en ce que** le collagène a été modifié par chauffage modéré à une température comprise entre 40 et 50°C.

15. Matériau collagénique selon l'une quelconque des revendications 1 à 14, **caracterisé en ce que** le collagène a subi un traitement de digestion à la pepsine.

16. Matériau collagénique selon l'une des revendications 1 à 5, 7 à 15, **caractérisée en ce que** le matériau est soumis à une irradiation de rayonnement béta.

17. Matériau collagénique selon la revendication 16, **caractérisé en ce que** le matériau est soumis à une irradiation béta à une dose supérieure ou égale à 25 kilogreys.

18. Procédé pour l'obtention d'un matériau collagénique biocompatible, non toxique, potentiellement adhérent à l'état humide et biodégradable en moins d'un mois, de préférence en moins d'une semaine,
**caractérisé en ce qu'**il comprend :
a) la préparation d'une solution de collagène;
b) le traitement de ladite solution pour faire perdre au collagène, au moins partiellement, sa structure hélicoïdale ;
c) le mélange de la solution collagénique résultante avec une solution contenant au moins un additif hydrophile macromoléculaire, chimiquement non réactif vis-à-vis du collagène présent ;
d) la réticulation dudit mélange pour obtenir le matériau collagénique désiré.

19. Procédé selon la revendication 18, **caractérisé en ce qu'**à l'étape a), on prépare une solution de collagène modifié par coupure oxydative.

20. Procédé selon la revendication 19, **caractérisé en ce que**, dans l'étape a) on prépare une solution acide de collagène, à une concentration comprise entre 5 et 50 g/l, et on la mélange à température ambiante, avec une solution d'acide périodique ou l'un de ses sels, à une concentration comprise entre 1 et 10⁻⁵ M.

21. Procédé selon l'une quelconque des revendications 18 à 20, **caractérisé en ce qu'**à l'étape b), on traite la solution de collagène par chauffage à une température supérieure à 37°C.

22. Procédé selon la revendication 21, **caractérisé en ce que**, dans l'étape b), on chauffe la solution de collagène à une température comprise entre 40 et 50° C.

23. Procédé selon l'une quelconque des revendications 18 à 22, **caractérisé en ce que** dans l'étape c), l'additif hydrophile présente un poids moléculaire supérieur à 3 000 Daltons.

24. Procédé selon l'une quelconque des revendications 18 à 23, **caractérisé en ce que** dans l'étape c), l'additif hydrophile est un polymère hydrophile de poids moléculaire compris entre 3 000 et 20 000 Daltons.

25. Procédé selon l'une quelconque des revendications 18 à 24, **caractérisé en ce que** dans l'étape c), l'additif hydrophile est le polyéthylène glycol.

26. Procédé selon l'une quelconque des revendications 18 à 25, **caractérisé en ce que** dans l'étape c), l'additif hydrophile est un polysaccharide choisi de préférence parmi l'amidon, le dextrane et la cellulose.

27. Procédé selon l'une quelconque des revendications 18 à 26, **caractérisé en ce que**, dans l'étape c), la concentration en additif hydrophile est de 2 à 10 fois inférieure à celle du collagène.

28. Procédé selon l'une quelconque des revendications 18 à 27, **caractérisé en ce que** dans l'étape c), on ajoute en outre de la glycérine.

29. Procédé selon la revendication 28, **caractérisé en ce que** la concentration en glycérine est comprise entre 3 et 8 g/l.

30. Procédé selon l'une quelconque des revendications 19 à 29, **caractérisé en ce qu'**à l'étape d), on neutralise le mélange jusqu'à pH neutre.

31. Procédé selon l'une quelconque des revendications 18 et 21 à 29, **caractérisé en ce qu'**à l'étape d), le mélange est réticulé par irradiation béta ou gamma.

32. Procédé selon l'une des revendications 18 à 30, **caractérisé en ce que** l'on soumet le matériau collagénique issu de l'étape d) à une irradiation de rayonnement béta.

33. Procédé selon la revendication 32, **caractérisé en ce que** le matériau collagénique est soumis à une irradiation béta à une dose supérieure ou égale à 25 kilogreys.

34. Procédé selon l'une quelconque des revendications 18 à 33, **caractérisé en ce que** l'on forme un film à partir de la solution contenant le collagène ayant perdu au moins partiellement sa structure hélicoïdale et éventuellement modifié par coupure oxydative, au moins un additif hydrophile et éventuellement de la glycérine.

35. Procédé selon la revendication 34, **caractérisé en ce que** l'on applique en couche mince la solution, de manière sensiblement uniforme, sur un support inerte sensiblement plan, on laisse s'effectuer la réticulation du collagène puis on sépare du support le film formé.

36. Procédé selon l'une quelconque des revendications 34 et 35, **caractérisé en ce que** la solution contient 2 à 6 % de collagène, 0,6 à 2 % d'additif hydrophile, 0,3 à 0,8 % de glycérine et la couche mince appliquée sur ledit support inerte présente une densité comprise entre 0,05 et 0,3 g/cm².

37. Procédé selon l'une quelconque des revendications 18 à 25, **caractérisé en ce que** l'on soumet le collagène réticulé à une incubation à 37°C en milieu humide, avant l'étape de stérilisation par irradiation, le matériau obtenu étant biodégradable en 2 à 4 semaines.

38. Procédé selon l'une quelconque des revendications 18 à 37, **caractérisé en ce que** le collagène de la solution de l'étape a) a subi un traitement de digestion à la pepsine.

39. Utilisation d'un matériau selon l'une quelconque des revendications 1 à 17, pour la préparation d'un film contre les adhérences post-opératoires.

## Claims

1. Biocompatible, non-toxic collagenic material which is biodegradable in less than one month, **characterised in that** it contains collagen and at least one macromolecular hydrophilic additive which is chemically unreactive with respect to collagen, the collagen having at least partially lost its helical structure and having undergone a crosslinking treatment, said material not being sticky to the touch in its dry state and being sticky in its moist state.

2. Collagenic material according to claim 1, **characterised in that** it is biodegradable in less than one week.

3. collagenic material according to one of claims 1 and 2, **characterised in that** the collagen has been modified by moderate heating to above 37°C.

4. Collagenic material according to one of claims 1 to 3, **characterised in that** it comprises collagen which has been modified by oxidative cleaving, crosslinked in the presence of at least one macromolecular hydrophilic additive which is chemically unreactive with respect to said collagen.

5. Collagenic material according to claim 4, **characterised in that** the collagen is modified by oxidative cleaving by means of a periodic acid or one of the salts thereof.

6. Collagenic material according to one of claims 1 to 3, **characterised in that** it is crosslinked by beta or gamma irradiation in the presence of a macromolecular hydrophilic additive which is chemically unreactive with respect to collagen.

7. Collagenic material according to any one of the preceding claims, **characterised in that** the macromolecular hydrophilic additive has a molecular weight of more than 3,000 Daltons.

8. Collagenic material according to claim 7, **characterised in that** the hydrophilic additive is a hydrophilic polymer with a molecular weight of between 3,000 and 20,000 Daltons.

9. Collagenic material according to any one of the preceding claims, **characterised in that** the hydrophilic additive is polyethyleneglycol.

10. Collagenic material according to any one of claims 1 to 7, **characterised in that** the hydrophilic additive is a polysaccharide preferably selected from among starch, dextran and cellulose.

11. Collagenic material according to any one of the preceding claims, **characterised in that** it contains collagen and at least one macromolecular hydrophilic additive in a ratio of collagen to hydrophilic additive(s) ranging from 1/1 to 9/1, preferably from 2/1 to 4/1 and more preferably 3/1.

12. Collagenic material according to any one of the preceding claims, **characterised in that** it further contains glycerol.

13. Collagenic material according to any one of the preceding claims, **characterised in that** it is in the form of a film.

14. Collagenic material according to any one of claims 1 to 13, **characterised in that** the collagen has been modified by moderate heating to a temperature of between 40 and 50°C.

15. Collagenic material according to any one of claims 1 to 14, **characterised in that** the collagen has undergone a treatment of digestion with pepsin.

16. Collagenic material according to one of claims 1 to 5 and 7 to 15, **characterised in that** the material is subjected to beta radiation.

17. Collagenic material according to claim 16, **characterised in that** the material is subjected to beta irradiation at a dosage greater than or equal to 25 kilogreys.

18. Process for obtaining a biocompatible non-toxic collagenic material which is potentially sticky in the moist state and is biodegradable in less than one month, preferably in less than a week, **characterised in that** it comprises:
a) preparing a collagen solution;
b) treating said solution to make the collagen lose its helical structure at least partially;
c) mixing the resulting collagenic solution with a solution containing at least one macromolecular hydrophilic additive which is chemically unreactive with respect to the collagen present;
d) crosslinking said mixture to obtain the desired collagenic material.

19. Process according to claim 18, **characterised in that** in step a) a modified collagen solution is prepared by oxidative cleaving.

20. Process according to claim 19, **characterised in that** in step a) an acid collagen solution is prepared at a concentration of between 5 and 50 g/l and this is mixed at ambient temperature with a solution of periodic acid or one of the salts thereof at a concentration of between 1 and 10⁻⁵ M.

21. Process according to any one of claims 18 to 20, **characterised in that** in step b) the collagen solution is treated by heating to a temperature above 37°C.

22. Process according to claim 21, **characterised in that** in step b) the collagen solution is heated to a temperature of between 40 and 50°C.

23. Process according to any one of claims 18 to 22, **characterised in that** in step c) the hydrophilic additive has a molecular weight of more than 3,000 Daltons.

24. Process according to any one of claims 18 to 23, **characterised in that** in step c) the hydrophilic additive is a hydrophilic polymer with a molecular weight of between 3,000 and 20,000 Daltons.

25. Process according to any one of claims 18 to 24, **characterised in that** in step c) the hydrophilic additive is polyethyleneglycol.

26. Process according to any one of claims 18 to 25, **characterised in that** in step c) the hydrophilic additive is a polysaccharide preferably selected from among starch, dextran and cellulose.

27. Process according to any one of claims 18 to 26, **characterised in that** in step c) the concentration of hydrophilic additive is 2 to 10 times less than that of the collagen.

28. Process according to any one of claims 18 to 27, **characterised in that** in step c) glycerol is also added.

29. Process according to claim 28, **characterised in that** the glycerol concentration is between 3 and 8 g/l.

30. Process according to any one of claims 19 to 29, **characterised in that** in step d) the mixture is neutralised to a neutral pH.

31. Process according to any one of claims 18 and 21 to 29, **characterised in that** in step d) the mixture is crosslinked by beta or gamma irradiation.

32. Process according to one of claims 18 to 30, **characterised in that** the collagenic material obtained in step d) is subjected to irradiation with beta ray.

33. Process according to claim 32, **characterised in that** the collagenic material is subjected to beta irradiation at a dosage greater than or equal to 25 kilogreys.

34. Process according to any one of claims 18 to 33, **characterised in that** a film is formed from the solution containing the collagen which has at least partially lost its helical structure and is optionally modified by oxidative cleaving, at least one hydrophilic additive and optionally glycerol.

35. Process according to claim 34, **characterised in that** the solution is applied in a substantially uniform manner in a thin layer to a substantially flat inert support, the collagen is allowed to crosslink and then the film formed is separated from the support.

36. Process according to any one of claims 34 and 35, **characterised in that** the solution contains 2 to 6% of collagen, 0.6 to 2% of hydrophilic additive, 0.3 to 0.8% of glycerol and the thin layer applied to said inert support has a density of between 0.05 and 0.03 g/cm².

37. Process according to any one of claims 18 to 25, **characterised in that** the crosslinked collagen is subjected to incubation at 37°C in a humid environment before the step of sterilisation by irradiation, the material obtained being biodegradable in 2 to 4 weeks.

38. Process according to any one of claims 18 to 37, **characterised in that** the collagen of the solution in step a) has undergone a treatment of digestion with pepsin.

39. Use of a material according to any one of claims 1 to 17 for preparing a film to combat post-operative adhesions.

## Patentansprüche

1. Collagenmaterial, das biologisch verträglich, nichttoxisch und in weniger als einem Monat biologisch abbaubar ist, **dadurch gekennzeichnet, dass** es Collagen und mindestens ein hydrophiles makromolekulares Additiv umfasst, das mit jenem chemisch nicht reaktiv ist, wobei das Collagen seine Helixstruktur wenigstens teilweise verloren hat und einer vernetzenden Behandlung unterworfen worden ist und dieses Material im trockenen Zustand bei Berührung nicht klebt und im feuchten Zustand haftfähig ist.

2. Collagenmaterial nach Anspruch 1, **dadurch gekennzeichnet, dass** es in weniger als einer Woche biologisch abbaubar ist.

3. Collagenmaterial nach Anspruch 1 oder 2, **dadurch gekennzeichnet, dass** das Collagen durch mäßiges Erwärmen auf über 37 °C modifiziert worden ist.

4. Collagenmaterial nach einem der Ansprüche 1 bis 3, **dadurch gekennzeichnet, dass** es Collagen umfasst, das durch oxidatives Schneiden modifiziert und in Gegenwart mindestens eines hydrophilen makromolekularen Additivs, das mit dem Collagen. chemisch nicht reaktiv ist, vernetzt worden ist.

5. Collagenmaterial nach Anspruch 4, **dadurch gekennzeichnet, dass** das Collagen durch oxidatives Schneiden mittels Periodsäure oder eines ihrer Salze modifiziert worden ist.

6. Collagenmaterial nach einem der Ansprüche 1 bis 3, **dadurch gekennzeichnet, dass** es in Gegenwart eines hydrophilen makromolekularen Additivs, das mit dem Collagen chemisch nicht reaktiv ist, durch β- oder γ-Strahlung vernetzt worden ist.

7. Collagenmaterial nach einem der vorhergehenden Ansprüche, **dadurch gekennzeichnet, dass** das hydrophile makromolekulare Additiv ein Molekulargewicht von über 3 000 Dalton aufweist.

8. Collagenmaterial nach Anspruch 7, **dadurch gekennzeichnet, dass** das hydrophile Additiv ein hydrophiles Polymer mit einem Molekulargewicht von 3 000 bis 20 000 Dalton ist.

9. Collagenmaterial nach einem der vorhergehenden Ansprüche, **dadurch gekennzeichnet, dass** das hydrophile Additiv Polyethylenglykol ist.

10. Collagenmaterial nach einem der Ansprüche 1 bis 7, **dadurch gekennzeichnet, dass** das hydrophile Additiv ein vorzugsweise aus Stärke, Dextran und Cellulose ausgewähltes Polysaccharid ist.

11. Collagenmaterial nach einem der vorhergehenden Ansprüche, **dadurch gekennzeichnet, dass** es Collagen und mindestens ein hydrophiles makromolekulares Additiv mit einem Verhältnis von Collagen/hydrophile(s) Additiv(e) von 1/1 bis 9/1, vorzugsweise 2/1 bis 4/1, und besonders bevorzugt von 3/1 umfasst.

12. Collagenmaterial nach einem der vorhergehenden Ansprüche, **dadurch gekennzeichnet, dass** es außerdem Glycerin enthält.

13. Collagenmaterial nach einem der vorhergehenden Ansprüche, **dadurch gekennzeichnet, dass** es in Form eines Films vorliegt.

14. Collagenmaterial nach einem der Ansprüche 1 bis 13, **dadurch gekennzeichnet, dass** das Collagen durch mäßiges Erwärmen auf eine Temperatur von 40 bis 50 °C modifiziert worden ist.

15. Collagermaterial nach einem der Ansprüche 1 bis 14, **dadurch gekennzeichnet, dass** das Collagen einer verdauenden Behandlung durch Pepsin unterworfen worden ist.

16. Collagenmaterial nach einem der Ansprüche 1 bis 5 und 7 bis 15, **dadurch gekennzeichnet, dass** dieses Material einer β-Strahlung ausgesetzt worden ist.

17. Collagenmaterial nach Anspruch 16, **dadurch gekennzeichnet, dass** dieses Material einer β-Strahlung mit einer Dosis von höher als oder gleich 25 kGray ausgesetzt worden ist.

18. Verfahren zur Herstellung eines im feuchten Zustand potentiell haftfähigen, nicht-toxischen biologisch verträglichen Collagenmaterials, das in weniger als einem Monat und vorzugsweise in weniger als einer Woche biologisch abbaubar ist, **dadurch gekennzeichnet, dass** es die
a) Herstellung einer Collagenlösung,
b) Behandlung dieser Lösung, damit das Collagen wenigstens teilweise seine Helixstruktur verliert,
c) Vermischung der resultierenden Collagenlösung mit einer Lösung, die mindestens ein hydrophiles makromolekulares Additiv, das mit dem vorhandenen Collagen chemisch nicht reaktiv ist, enthält, und
d) Vernetzung dieses Gemischs, um das gewünschte Collagenmaterial zu erhalten,
umfasst.

19. Verfahren nach Anspruch 18, **dadurch gekennzeichnet, dass** in Stufe a) eine Lösung des Collagens hergestellt wird, das durch oxidatives Schneiden modifiziert worden ist.

20. Verfahren nach Anspruch 19, **dadurch gekennzeichnet, dass** in Stufe a) eine saure Collagenlösung mit einer Konzentration von 5 bis 50 g/l hergestellt und bei Umgebungstemperatur mit einer Lösung von Periodsäure oder einem ihrer Salze mit einer Konzentration von 1 bis 10⁻⁵ m vermischt wird.

21. Verfahren nach einem der Ansprüche 18 bis 20, **dadurch gekennzeichnet, dass** die Collagenlösung in Stufe b) durch Erwärmen auf eine Temperatur von über 37 °C behandelt wird.

22. Verfahren nach Anspruch 21, **dadurch gekennzeichnet, dass** die Collagenlösung in Stufe b) auf eine Temperatur von 40 bis 50 °C erwärmt wird.

23. Verfahren nach einem der Ansprüche 18 bis 22, **dadurch gekennzeichnet, dass** das hydrophile Additiv in Stufe c) ein Molekulargewicht von über 3 000 Dalton aufweist.

24. Verfahren nach einem der Ansprüche 18 bis 23, **dadurch gekennzeichnet, dass** das hydrophile Additiv in Stufe c) ein hydrophiles Polymer mit einem Molekulargewicht von 3 000 bis 20 000 Dalton ist.

25. Verfahren nach einem der Ansprüche 18 bis 24, **dadurch gekennzeichnet, dass** das hydrophile Additiv in Stufe c) Polyethylenglykol ist.

26. Verfahren nach einem der Ansprüche 18 bis 25, **dadurch gekennzeichnet, dass** das hydrophile Additiv in Stufe c) ein vorzugsweise aus Stärke, Dextran und Cellulose ausgewähltes Polysaccharid ist.

27. Verfahren nach einem der Ansprüche 18 bis 26, **dadurch gekennzeichnet, dass** die Konzentration des hydrophilen Additivs in Stufe c) 2 bis 10 Mal niedriger als die des Collagens ist.

28. Verfahren nach einem der Ansprüche 18 bis 27, **dadurch gekennzeichnet, dass** in Stufe c) außerdem Glycerin zugegeben wird.

29. Verfahren nach Anspruch 28, **dadurch gekennzeichnet, dass** die Glycerinkonzentration 3 bis 8 g/l beträgt.

30. Verfahren nach einem der Ansprüche 19 bis 29, **dadurch gekennzeichnet, dass** das Gemisch in Stufe d) bis auf den neutralen pH-Wert neutralisiert wird,

31. Verfahren nach einem der Ansprüche 18 und 21 bis 29, **dadurch gekennzeichnet, dass** das Gemisch in Stufe d) durch β- oder γ-Strahlung vernetzt wird.

32. Verfahren nach einem der Ansprüche 18 bis 30, **dadurch gekennzeichnet, dass** das aus Stufe d) stammende Collagenmaterial einer β-Strahlung ausgesetzt wird.

33. Verfahren nach Anspruch 32, **dadurch gekennzeichnet, dass** das Collagenmaterial einer β-Strahlung mit einer Dosis von höher als oder gleich 25 kGray ausgesetzt wird.

34. Verfahren nach einem der Ansprüche 18 bis 33, **dadurch gekennzeichnet, dass** aus der Lösung, die das Collagen, das seine Helixstruktur wenigstens teilweise verloren hat und gegebenenfalls durch oxidatives Schneiden modifiziert worden ist, mindestens ein hydrophiles Additiv und gegebenenfalls Glycerin enthält, ein Film gebildet wird.

35. Verfahren nach Anspruch 34, **dadurch gekennzeichnet, dass** die Lösung in Form einer dünnen Schicht im Wesentlichen gleichmäßig auf einen im Wesentlichen ebenen inerten Träger aufgebracht, das Collagen sich vernetzen gelassen und anschließend der gebildete Film vom Träger abgezogen wird.

36. Verfahren nach Anspruch 34 oder 35, **dadurch gekennzeichnet, dass** die Lösung 2 bis 6 % Collagen, 0,6 bis 2 % hydrophiles Additiv und 0,3 bis 0,8 % Glycerin enthält und die auf dem inerten Träger aufgebrachte dünne Schicht ein Flächengewicht von 0,05 bis 0,3 g/cm² besitzt.

37. Verfahren nach einem der Ansprüche 18 bis 25, **dadurch gekennzeichnet, dass** das vernetzte Collagen vor der Stufe der Sterilisation durch Bestrahlung einer Inkubation bei 37 °C im nassen Medium unterworfen wird, wobei das erhaltene Material innerhalb von 2 bis 4 Wochen biologisch abbaubar ist.

38. Verfahren nach einem der Ansprüche 18 bis 37, **dadurch gekennzeichnet, dass** das Collagen der Lösung von Stufe a) einer verdauenden Behandlung durch Pepsin unterworfen worden ist.

39. Verwendung eines Materials nach einem der Ansprüche 1 bis 17 zur Herstellung eines Films gegen postoperative Adhäsionen.
